# EUROPEAN PATENT APPLICATION

(11) **EP 1 479 799 A1**
(43) Date of publication of application: **24.11.2004**
(21) Application number: 02743861.3
(22) Date of filing: 08.07.2002
(51) Int. Cl.: D01F 9/00, C07H 15/20

(54) **FIBROUS NANO SELF-ASSEMBLIES**

(30) Priority: 26.02.2002 JP 2002049239
(71) Applicant: Japan Science and Technology Agency, Kawaguchi-shi, Saitama 332-0012 (JP); National Institute of Advanced Industrial Science and Technology, Tokyo 100-8921 (JP)
(72) Inventor: SHIMIZU, Toshimi Tsukuba Center, Tsukuba-shi, Ibaraki 305-8561 (JP); JOHN, George, Tsukuba-shi, Ibaraki 305-8561 (JP)
(74) Representative: Wilson Gunn Gee
(86) International application number: PCT/JP2002/006923
(87) International publication number: WO 2003/072858

(57) **Abstract**

A means to continuously and freely control morphology ranging from twisted ribbon construction to tubular shape by optionally changing the mix ratio of multiple numbers of synthetic oligolipid components and a fibrous nanoscale self-aggregate construction morphology which is controlled in this manner are presented.

The present invention is a fibrous nanoscale self-aggregate comprising O-glycoside type oligolipid represented by the general formula below (Chemical Formula 1) 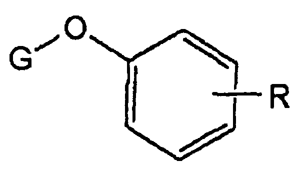 (wherein G represents a glycosyl group and R represents a hydrocarbon group containing 12 to 18 carbon atoms), wherein said O-glycoside type oligolipid comprises at least two kinds of O-glycoside type oligolipids having different said structure, wherein the proportion of the two major kinds of said O-glycoside type oligolipid being at least 80% by weight of said O-glycoside type oligolipid, or comprises an O-glycoside type oligolipid having one type of said structure.

## Description

### Technical field of the invention

This invention relates to a fibrous nanoscale self-aggregate formed by a voluntary aggregation of lipid molecules, having size dimensions constituting a thickness of several tens of nm, a width of several tens to several hundreds of nm and a length of several tens to several hundreds of µm and having a high aspect ratio. The present invention further relates to a manufacturing process and an unrestricted morphology regulation process for various helical nanoscale self-aggregates and lipid nanotubes with potential utility in fields such as fine chemicals, pharmaceutical products, cosmetics, electronic data, the energy industry and chemical manufacturing.

### Background of the prior art

Some lipids self aggregate in water to form stable molecular aggregates having various morphologies such as spherical, band-like, rod-like, granular and disk-like shapes and are used as functional materials in the fields of fine chemicals and medicine. (For example, see Toyoki Kunitake, Comprehensive Supramolecular Chemistry, 1996, Vol. 9, p. 351.) However, the self-aggregate morphology obtained is determined by the structure of the constituent elements comprising the lipid molecular structure and the balance among various intermolecular interactions greatly influenced by the same. A serious problem encountered was that optional and complete aggregate morphology regulation was dependent on an empirical process wherein many more lipid molecules were synthesized and a library of self-aggregate morphologies was created. Furthermore, the molecular aggregates obtained according to these methods consisted almost entirely of spherical shapes, and fibrous molecular aggregates having a large aspect ratio, that is the ratio of fiber length to width, were difficult to obtain. A restricted field of utility was an inescapable consequence of the situation.

Although not many examples are known, fibrous or rod shaped molecular aggregates are obtained when synthetic amphiphilic compounds are dispersed in water. ["Journal of the American Chemical Society", Vol. 107, pp. 509-510 (1985).]

However, the molecular aggregates obtained according to that method were either in a ribbon-shaped or string-shaped simple self-aggregate morphology and could not be optionally prepared in a nanotube morphology having one dimensional independent voids and large surface area or in a coil shaped ribbon morphology effective in trapping gas or separating useful biological molecules. These molecular aggregates were not almost totally useful in the form of fibrous molecular aggregates.

### Problems encountered

The objective of the present invention is to present a means for continuous morphology regulation wherein the morphology can be changed from a twisted ribbon construction to loosely coiled, tightly coiled and tubular shapes by simply changing the mixing ratio of multiple synthetic glycolipid components that can be easily synthesized and separated using readily available and inexpensive natural resources, and a fibrous nanoscale self-aggregated wherein the morphology being regulated by said means.

### Means to Solve the Problems

The inventors have been diligently researching glycolipid related compounds capable of self-assembly without undergoing phase separation from each other that may be obtained from plant based starting materials that are readily available and renewable. As a result, a combination of several components in the o-glycoside type glycolipid component obtained as an aglycon of various components from the long chain alkyl phenol mixture separated from cashew nutshell oil was allowed to self-aggregate. The research revealed that the helical ribbon morphology could be continuously controlled from twisted to tubular morphology, and the present invention was completed based on this acquired knowledge.

That is, the present invention is a fibrous nanoscale self-aggregate comprising O-glycoside type oligolipid represented by the general formula below (Chemical Formula 1) 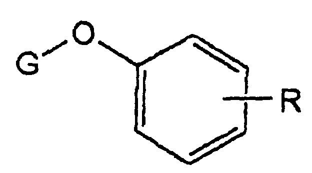 (wherein G represents a glycosyl group and R represents a hydrocarbon group containing 12 to 18 carbon atoms), wherein said O-glycoside type oligolipid comprises at least two kinds of O-glycoside type oligolipids having different said structure, wherein the proportion of the two major kinds of said O-glycoside type oligolipid being at least 80% by weight of said O-glycoside type oligolipid, or comprises an O-glycoside type oligolipid having one type of said structure.

In addition, the present invention is a process for producing a fibrous nanoscale self-aggregate comprising an O-glycoside type oligolipid having a structure represented by the general formula below (Chemical Formula 1) 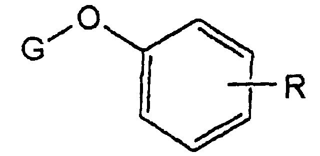 (wherein G represents a glycosyl group and R represents a hydrocarbon group containing 12 to 18 carbon atoms) which comprises a step of dispersing O-glycoside type oligolipid in an aqueous medium, wherein said O-glycoside type oligolipid comprises at least two kinds of O-glycoside type oligolipids having different said structure, wherein the proportion of the two major kinds of said O-glycoside type oligolipid being at least 80% by weight of said O-glycoside type oligolipid, or comprises an O-glycoside type oligolipid having one type of said structure.

The proportion of the aforementioned two main o-glycoside type glycolipids is, based on the weight of said o-glycoside type glycolipids, preferably at least 90% by weight, more preferably 100% by weight, that is, the aforementioned o-glycoside type glycolipid being a mixture of two o-glycoside type glycolipids. Furthermore, the aforementioned at least two o-glycoside type glycolipids having different aforementioned hydrocarbon groups is preferred, the degree of saturation of the aforementioned hydrocarbon groups being different is more preferred and the degree of saturation of the aforementioned hydrocarbon groups being saturated or mono-ene is particularly preferred. As mentioned above, the structure described by the general formula (Chemical Formula 1) of the o-glycoside type glycolipidmaybe a single structure, and in this case a saturated or mono-ene structure is preferred for the hydrocarbon (R in the formula).

### Brief Description of the Drawings

Figure 1 shows a 1H-NMR spectrum (600 MHz in deuterated methanol) of a saturated component of the components of the cardanol type glycolipid obtained in Production Example 2.
Figure 2 shows a 1H-NMR spectrum (600 MHz in deuterated methanol) of a mono-ene type component of the components of the cardanol type glycolipid obtained in Production Example 2.
Figure 3 shows a 1H-NMR spectrum (600 MHz in deuterated methanol) of a diene type component of the components of the cardanol type glycolipid obtained in Production Example 2.
Figure 4 shows a 1H-NMR spectrum (600 MHz in deuterated methanol) of a triene type component of the components of the cardanol type glycolipid obtained in Production Example 2.
Figure 5 shows a photograph of the fibrous nanoscale self-aggregates having various helical morphologies obtained in Examples 1-7 and Reference Example 1. The arrows in figures (b) to (f) indicate twisted sections, and the arrow in figure (h) shows a tube cross section.
Figure 6 shows changes in fibrous nanoscale self-aggregate morphology when the aggregate has various morphologies. (1) corresponds to Figure 5 a, b and c (Examples 1-3), (2) corresponds to Figure 5 d (Example 4), (3) and (4) correspond to Figure 5 e (Example 5), (5) corresponds to Figure 5 f (Example.6), and (6) corresponds to Figure 5 g (Example 7) and Figure 5 h (Reference Example 1).

### Description of the Preferred Embodiment

A surface activating organic compound used in the present invention is an o-glycoside type glycolipid represented by the general formula (Chemical Formula 1) shown below.

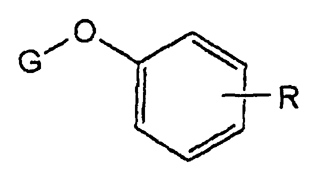

In the present invention, the hydrocarbon group (R) may be located at o-, m- or p-position to the -O-G group, but the meta (m-) position is preferred.
The G in the aforementioned general formula represents a glycosyl group, and radicals obtained by removing the hydrogen atom from the reducing terminal hydroxyl group of aldopyranoses, such as glucopyranose, galactopyranose, mannopyranose, allopyranose, altropyranose, gulopyranose, idopyranose and talopyranose, or corresponding aldofuranose, for example, can be mentioned.

In addition, the R in the aforementioned general formula is a hydrocarbon group containing twelve to eighteen carbon atoms and preferably is an aliphatic hydrocarbon comprising saturated and/or unsaturated aliphatic hydrocarbons. This hydrocarbon is preferably linear. In addition, the number of carbon atoms in this hydrocarbon is preferably fourteen to sixteen, more preferably fifteen. As such hydrocarbon groups, dodecyl group, tridecyl group, tetradecyl group, pentadecyl group, hexadecyl group, heptadecyl group, octadecyl group and groups of the same containing a monoene, a diene, or a triene and the like, for example, can be mentioned. Among these, 8-pentadecyl group, 8,10-pentadecadienyl group and 8,10,12-pentadecatrienyl group are preferred due to ready availability of the raw material.

Such o-glycoside type oligolipids can be produced, for example, by allowing a long chain alkyl phenol represented by a general formula (Chemical Formula 2) 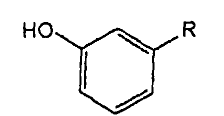 (an example is a compound wherein R is an alkyl group and R is located in the m-position to the OH) to react with the reducing terminal hydroxyl group of a reactive functional derivative of aldopyranose or aldofuranose (henceforth simply referred to as protected aldose) wherein all hydroxyl groups other than the reducing terminal hydroxyl group are protected to form an o-glycoside bond and subsequently removing the protective groups. Acetyl, benzyl and 1,2-isopropylidene groups, for example, may be used as the protective groups,.
An alkyl group having a desired structure within the scope specified above is selected as the long chain alkyl group (R) in the formula and is converted into an alkyl phenol [general formula (Chemical Formula 2)] according to an established method.

As the reactive functional derivative of a reducing terminal hydroxyl group, the corresponding aldose trichloroacetoimidate, bromide (bromo sugar), fluoride (fluoro sugar), thioglycoside and o-acylate, for example, can be mentioned. Of these, the fluoride and trichloroacetoimidate are preferred due to the high yield reactions obtained.

The reactive functional derivative of a protected aldose allowed to react with the long chain alkyl phenol mixture can be produced, for example, as described below.

That is, halides such as bromides or fluorides, so-called bromo sugars or fluoro sugars, of an aldose reducing terminal hydroxyl group can be obtained by acetylating aldose in pyridine and then allowing the product to react with hydrogen bromide or hydrogen fluoride in acetic acid.

In addition, a corresponding trichloroacetoimidate can be obtained by similarly acetylating aldose as described above first, subsequently allowing the product to react with hydrazine acetic acid salt in dimethyl formamide to form an oligo chain component only the reducing terminals of which are selectively de-acetylated and next allowing said oligo chain component to react with trichloroacetonitrile in the presence of a base catalyst. As the reaction solvent for this case, halogenated compounds such as methylene chloride and chloroform are preferred. In addition, as the base catalyst, sodium hydride, cesium carbonate and the like are preferred.

In the reaction used to obtain a halogenated aldose, the α-isomer is selectively obtained. In the reaction used to obtain a trichloroacetoimidate, the α-isomer can be selectively obtained when the reaction is allowed to proceed for at least two hours at room temperature. The results can be confirmed by the fact that a doublet signal (spin-spin coupling constant = 3.4-4.0 Hz) having a δ value of 6.4-6.6 ppm in 1H-NMR spectrum (in deuterated chloroform at 25 degree C) is observed.

Next, the reaction used to form an o-glycoside bond from a mixture of long chain alkyl phenols represented by general formula (Chemical Formula 2) and a protected aldose reactive functional derivative can be conducted as described below. When the protected aldose reactive functional derivative is a bromide, for example, the reaction is allowed to occur in the presence of a basic substance using tin trifluoromethane sulfonate as the catalyst. Chloroform, toluene and the like can be used as the reaction solvent for this example, but a chloroform/toluene mixed solvent system is preferred from the standpoint of solubility. In addition, 2,4,6-trimethylpyridine and 1,1,3,3-tetramethyl urea can be used as the basic substance. A reaction temperature for this example of from room temperature to 40°C for ten to twenty hours is appropriate. An even better yield is obtained in this reaction when molecular sieve 4A is co-present.

Next, a Lewis acid catalyst is used when the protected aldose reactive functional derivative is trichloroacetoimidate. Halogenated solvents such as chloroform, methylene chloride, 1,2-dichloroethane and the like, acetonitrile, nitromethane and the like can be used as the reaction solvent. Methylene chloride is particularly preferred. As the Lewis acid catalyst for this reaction, trimethylsilyl trifluoromethane sulfonate and boron trifluoride ether complex can be used. Two to three equivalents per trichloroacetoimidate is ideal as the amount of Lewis acid catalyst used. A reaction temperature for this occasion of -5 to 0 degree C is appropriate. The reaction time is ordinarily two to three hours although it is influenced by the type of Lewis acid catalyst and the reaction temperature. This reaction is preferably conducted in the presence of molecular sieve with agitation. An o-glycoside can be obtained in good yield when glucose is used as an aldose and boron trifluoride ether complex is used as the catalyst even when the glucose is not first converted into a trichloroacetoimidate and all of the hydroxyl groups including reducing terminal hydroxyl groups are allowed to react directly with the aldose. The use of glucose is particularly convenient since this method results in high yield reactions. The o-glucoside β-isomer is selectively obtained when a bromide or trichloroacetoimidate is used. The compounds can be confirmed by the fact that a doublet signal (spin-spin coupling constant = 7.8-8.0 Hz) having a δ value of 4.4-4.9 ppm in 1H-NMR spectrum (in deuterated chloroform at 25 degree C) is observed.

The protective groups of o-glycoside type oligolipids containing protected aldose radicals obtained in the manner described need to be released as a final step.

A reaction to release protective groups such as, for example, acetyl groups can be conducted by treating a protected o-glycoside with an alkali metal alcoholate such as sodiummethoxide or potassium methoxide followed by neutralization using a strongly acidic cation exchange resin. In addition, the treatment can be conducted more simply by mixing an aqueous solution of a trialkyl amine such as trimethylamine with a several fold volumetric excess of a reaction solvent to allow the o-glycoside having the aforementioned protected oligo chains to react. The concentration of an aqueous trialkylamine solution at this point of 30% by weight to 50% by weight is preferred. Alcohol type solvents such as methyl alcohol, ethyl alcohol and the like and mixed solvents of ether type solvents such as diethyl ether, tetrahydrofuran and the like with alcohol type solvents are appropriate for the reaction solvent for this occasion. Maintaining the reaction solution pH at 8.0 to 8.5 for this occasion is desirable from the standpoint of avoiding side reactions such as ester hydrolysis. The reaction time is influenced by the reaction conditions, but twelve to 24 hours is ordinarily appropriate. Upon completion of the process, o-glycoside type oligolipids containing long chain alkyl phenol radicals represented by the aforementioned general formula (Chemical Formula 1) as aglycons are obtained in the form of white powders. A crude product obtained in this manner can be converted into a high purity material using a silica gel column separation purification operation.

The o-glycoside type oligolipid mixtures obtained in this manner have experimental elemental analysis results that agree with the calculated values within an error margin. Furthermore, a compound containing oligo chains protected by acetyl groups can readily be identified from the characteristic signals attributed to the methyl hydrogens in the acetyl groups having a δ value of 2.03 to 2.08 ppm in an 1H-NMR spectrum (in deuterated chloroform at 25°C). On the other hand, a compound from which acetyl groups had been removed can be identified and confirmed with its δ value at 0.88 ppm (the methyl group hydrogens in a long chain alkyl group), 1.26 ppm (the methylene group hydrogens in a long chain alkyl group) , 1.58 ppm (the second methylene group hydrogens in a long chain alkyl group counting from the aromatic section), 2.56 ppm (the methylene group hydrogens directly connected to the aromatic group) , 3.13-3.69 ppm (the hydrogens connected to the C2, C3, C4, C5 and C6 carbon atoms in the oligo chain), 4.82 ppm (the anomer hydrogen connected to the C1 carbon in the oligo chain), 5.34-5.42 ppm (the hydrogen connected to the vinyl group), 6.79 ppm, 6.80-6.89 ppm and 7.19-7.20 ppm (the hydrogen connected to the aromatic ring).

In the present invention, no restrictions apply to the production process for fibrous nanoscaleself-aggregates. However, the aggregates can be obtained by dispersing the o-glycoside type oligolipid mentioned above in water, subsequently heating the dispersion using a mantle heater, boiling for about twenty minutes, allowing the mixture to cool to room temperature naturally and by allowing it to stand at room temperature until fibrous nanoscale self-aggregates are formed. (Japanese Patent Applications 2000-271192 and 2001-363762)

The fibrous nanoscale self-aggregate production process of the present invention is described in further detail. A multiple number or a single type of o-glycoside type oligolipid compounds appropriately selected are mixed in a desired weight ratio and are dissolved in an organic solvent. Alcohol type solvents such as methanol, ethanol and the like are ideal based on solubility. Upon dissolution, the solvent is allowed to evaporate and perfectly mixed multicomponent solids are obtained. A saturated dispersion is prepared by adding water to the solids and heating the mixture to a boil. When the amount of water is too little at this point, an insoluble fraction remains. When the amount of water is too great, a saturated concentration is not reached. Therefore, the amount of water added is selected from a range of from twenty to 1,000 fold excess by weight based on o-glycoside. A heating temperature at this point that allows the mixture to reflux for about an hour at the boiling temperature is preferred in order to maximize the amount of o-glycoside dissolved. However, a dispersion can also be prepared if desired by sonicating the mixture at lower temperatures.

Next, the saturated aqueous o-glycoside prepared was gradually cooled and was allowed to stand at room temperature to form various fibrous nanoscale self-aggregates. Long fibers are difficult to obtain when the cooling rate at this point are too rapid, and short fibers of the aggregates are formed. Therefore, the selection of a cooling rate of 0.5 degree C/minute or slower is preferred, and a range of 0. 2 degree C/minute or slower is particularly preferred. Water alone is ordinarily used as the solvent to prepare aqueous solutions. After one to two days of gradual cooling in the manner described, a fibrous substance separates from the aqueous solution. The fibrous self-aggregate nanoscale structure material is collected and air dried or dried in vacuum to obtain a helical self-aggregated nanoscale structured material that is stable in air and has dimensions of several tens of nm in thickness, several tens to several hundreds of nm in width and several tens to several hundreds of µm in length.

The presence of the fibrous structured material obtained can be easily observed using an ordinary optical microscope. However, the detailed helical self-aggregated morphology can be clearly confirmed using an electron microscope.

A fibrous nanoscale self-aggregate obtained according to the process of the present invention can be utilized in the fine chemicals industry as a material used to capture and separate pharmaceutical agents and useful biomolecules in the pharmaceutical and cosmetic fields, as a drug delivery material or as microelectric parts in electronic and data fields upon coating the nanotubes with electroconductive substances and metals. Furthermore, its usefulness in the energy industry as a material to absorb and store various gases and as a catalyst support and also in the medical, analytical and chemical production fields as artificial blood vessels, nanotube capillaries and nano-reactors that utilize the fine tubular construction make its industrial utility value enormous.

The following examples illustrate the invention without however limiting it. The Rf values for thin layer chromatography reported below are the values obtained when a mixed hexane/ethyl acetate solvent (volume ratio 6/4) was used as the developing solvent.

### Production Example 1

Cashew nutshell oil was distilled twice under vacuum at about 400 Pa, and the fraction boiling at 220 to 235 degree C was collected to obtain cardanol. Five millimoles (1.52 g) of the cardanol was dissolved in 10 ml of anhydrous methylene chloride, and 3.9 g (five millimoles) β-D-glucose pentaacetate and 0.62 ml (five millimoles) of boron trifluoride diethyl ether were added in the presence of two grams of a molecular sieve. The reaction mixture was agitated for 24 hours and was poured into a 5% aqueous sodium bicarbonate solution. The organic phase was separated and was consecutively washed using an aqueous sodium bicarbonate solution and water. The organic phase was dried over anhydrous sodium sulfate. The organic solvent was completely removed by distillation under reduced pressure, and the crude product obtained was recrystallized from ethanol. The solid product obtained was purified using column chromatography using a hexane/ethyl acetate (volume ratio 7/3) mixed solvent as the elusion solution to yield 2.36 g (75% yield) of 1-(O-β-D-glucopyranoside tetraacetate) in the form of white solids.
The physical properties of the product were as shown below.
The Rf value in thin layer chromatography: Rf1 = 0.47
Melting point: 60 degree C

| Elemental analysis (C₃₅H₅₀O₁₀) | | |
|---|---|---|
| | C | H |
| Theoretical (%) | 66.65 | 7.99 |
| Experimental (%) | 66.78 | 7.82 |

Next, a 45% by weight aqueous trimethylamine solution was mixed with four times the volume of methanol, and the mixture was allowed to react for 24 hours with the 1-(O-β-D-glucopyranoside tetraacetate) cardanol obtained. The solvent was removed by distillation under reduced pressure, the syrup-like residue obtained was allowed to crystallize from a methanol/acetonitrile (volume ratio ½) mixed solvent and was further recrystallized from the same solvent to obtain almost quantitatively 0.88 g (95% yield) of the desired de-acetylated white solids 1- (O-β-D-glucopyranoside tetraacetate) cardanol.
The physical properties of the product were as shown below.
Melting point: 135.2 degree C

| Elemental analysis (C₂₇H₄₂O₆) | | |
|---|---|---|
| | C | H |
| Theoretical (%) | 70.10 | 9.15 |
| Experimental (%) | 70.39 | 9.44 |

### Production Example 2

Next, the O-glycoside type oligolipid mixture synthesized in this manner was separated into four types of constitutional components.

The cardanol obtained by extracting cashew nut shell oil used as the raw material is generally known to contain long chain alkylphenols containing at position three a pentadecyl group (saturated), an 8-pentadecenyl group (monoene), an 8,11-pentadecadienyl group (diene) and an 8,11,14-pentadecatrienyl group (triene) in amounts of about 5%, about 50%, about 16% and about 29% (published values), respectively.

Column chromatography capable of separating the desired components when a sample solution containing a mixture is added dropwise to a column packed with a suitable filler and discharged using a suitable elution solution was used for the purification and separation. In order to make the separation performance and separation time more efficient, a medium pressure chromatography device packed with a reverse phase type silica gel filler and a mixed solvent system (volume ratio 88:12) of methanol and 10% acetic acid were used to realize ideal conditions.

Column chromatography was executed using 300 mg of the deacylated 1-(O-β-D-glucopyranoside) cardanol and a reverse phase silica gel (particle size: 50 microns) packed into a medium pressure column (100 cm long x 2.6 cm inner diameter). A mixed solvent system of methanol and 10% acetic acid was used as the elution solution. The volume ratio was gradually changed from 90:10 to 88:12. The flow rate was 8 ml/minute, and the compounds were detected using the degree of absorption at 254 nm. As a result, about 7 mg of the saturated component, about 70 mg of the monoene component, 10 mg of the diene component and about 30 mg of the triene component were collected. The pure O-glycoside type oligo lipid obtained in this manner had elemental analysis results agreeing with the theoretical values within the margin of error.
The physical properties of the saturated component were as shown below.
Melting point: 143.6 degree C

| Elemental analysis (C₂₇H₄₆O₆) | | |
|---|---|---|
| | C | H |
| Theoretical (%) | 69.49 | 9.94 |
| Experimental (%) | 68.99 | 9.88 |

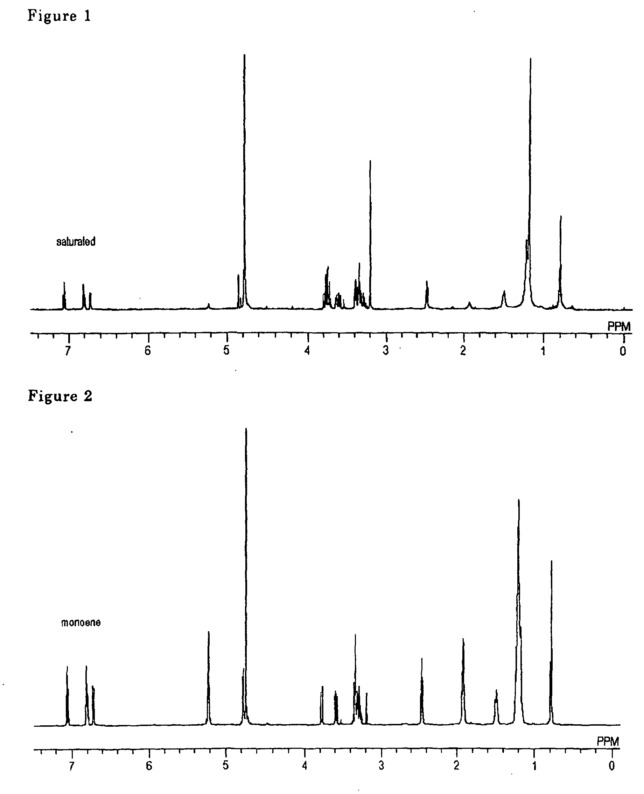

Furthermore, the saturated component was identified and confirmed from the products showing signals in 1H-NMR spectrum (in deuterated methanol at 25°C) at δ value of 0.88 ppm (the methyl group hydrogens in a long chain alkyl group), 1.26 ppm (the methylene group hydrogens in a long chain alkyl group), 1.58 ppm (the second methylene group hydrogens in a long chain alkyl group counting from the aromatic section), 2. 56 ppm (the methylene group hydrogens directly connected to the aromatic group), 3.13-3.69 ppm (the hydrogens connected to the C2, C3, C4, C5 and C6 carbon atoms in the oligo chain), 4.82 ppm (the anomer hydrogen connected to the C1 carbon in the oligo chain), 6.79, 6.80-6.89 ppm and 7.19-7.20 ppm (the hydrogen connected to the aromatic ring). The 1H-NMR spectrum of the saturated component obtained is shown in Figure 1.
The physical properties of the monoene component were as shown below.
Melting point: 132 degree C

| Elemental analysis (C₂₇H₄₄O₆) | | |
|---|---|---|
| | C | H |
| Theoretical (%) | 69.82 | 9.48 |
| Experimental (%) | 69.41 | 9.46 |

Furthermore, the monoene type component was identified and confirmed from the products showing signals in 1H-NMR spectrum (in deuterated methanol at 25 degree C) at δ value of 0.88 ppm (the methyl group hydrogens in a long chain alkyl group), 1.25 ppm (the methylene group hydrogens in a long chain alkyl group), 1.58 ppm (the second methylene group hydrogens in a long chain alkyl group counting from the aromatic section), 2.00 ppm (the methylene group hydrogen connected to the vinyl group), 2.56 ppm (the methylene group hydrogens directly connected to the aromatic group), 3.13-3.69 ppm (the hydrogens connected to the C2, C3, C4, C5 and C6 carbon atoms in the oligo chain), 4.82 ppm (the anomer hydrogen connected to the C1 carbon in the oligo chain), 5.3 ppm (the vinyl group hydrogen), 6.79, 6.80-6.89 ppm and 7.19-7.20 ppm (the hydrogen connected to the aromatic ring). The ¹H-NMR spectrum of the monoene type component obtained is shown in Figure 2.
The physical properties of the monoene component are as shown below.
Melting point: 132 degree C

| Elemental analysis (C₂₇H₄₄O₆) | | |
|---|---|---|
| | C | H |
| Theoretical (%) | 69.82 | 9.48 |
| Experimental (%) | 69.41 | 9.46 |

Furthermore, a monoene type component can be identified and confirmed from the products showing signals in 1H-NMR spectrum (in deuterated methanol at 25 dgree C) at δ value of 0.88 ppm (the methyl group hydrogens in a long chain alkyl group), 1.25 ppm (the methylene group hydrogens in a long chain alkyl group), 1.58 ppm (the second methylene group hydrogens in a long chain alkyl group counting from the aromatic section), 2.00 ppm (the methylene group hydrogen connected to the vinyl group), 2.56 ppm (the methylene group hydrogens directly connected to the aromatic group) , 3.13-3.69 ppm (the hydrogens connected to the C2, C3, C4, C5 and C6 carbon atoms in the oligo chain) , 4.82 ppm (the anomer hydrogen connected to the C1 carbon in the oligo chain), 5.3 ppm (the vinyl group hydrogen), 6.79, 6.80-6.89 ppm and 7.19-7.20 ppm (the hydrogen connected to the aromatic ring). The ¹H-NMR spectrum of the monoene type component obtained is shown in Figure 2.

The physical properties of the diene type component were as shown below.
Melting point: 115 degree C

| Elemental analysis (C₂₇H₄₂O₆) | | |
|---|---|---|
| | C | H |
| Theoretical (%) | 70.12 | 9.09 |
| Experimental (%) | 69.94 | 8.97 |

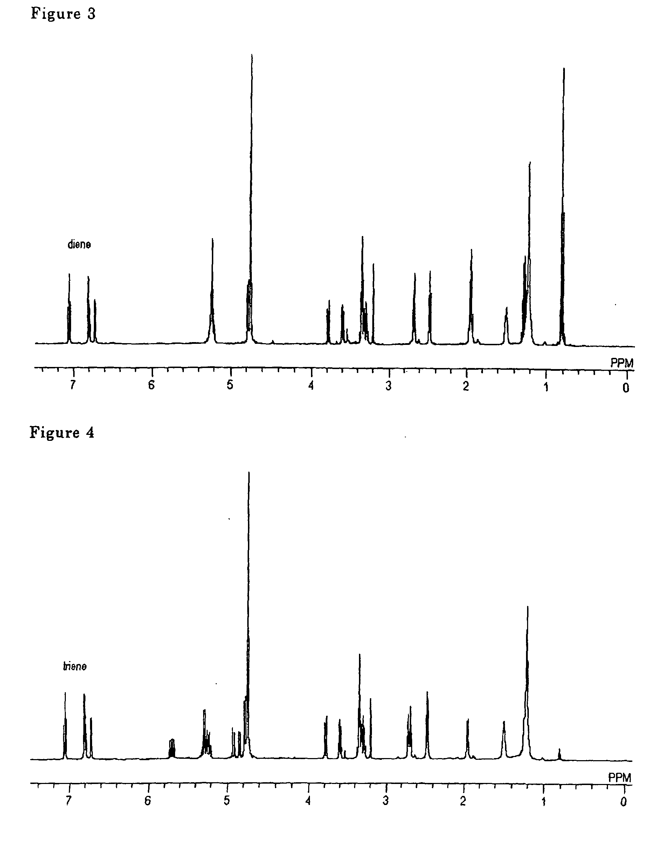

Furthermore, the diene type component was identified and confirmed from the products showing signals in ¹H-NMR spectrum (in deuterated methanol at 25 degree C) at δ value of 0.88 ppm (the methyl group hydrogens in a long chain alkyl group), 1.25 ppm (the methylene group hydrogens in a long chain alkyl group), 1.58 ppm (the second methylene group hydrogens in a long chain alkyl group counting from the aromatic section), 2.00 ppm (the methylene group hydrogen connected to the vinyl group), 2.56 ppm (the methylene group hydrogens directly connected to the aromatic group), 2.78 ppm (the methylene group hydrogens sandwiched between two vinyl groups), 3.13-3.69 ppm (the hydrogens connected to the C2, C3, C4, C5 and C6 carbon atoms in the oligo chain), 4.82 ppm (the anomer hydrogen connected to the C1 carbon in the oligo chain) , 5. 3 ppm (the vinyl group hydrogen) , 6.79, 6.80-6.89 ppm and 7.19-7.20 ppm (the hydrogen connected to the aromatic ring). The ¹H-NMR spectrum of the monoene type component obtained is shown in Figure 3.

The physical properties of the triene component were as shown below.
Melting point: 96 degree C

| Elemental analysis (C₂₇H₄₀O₆) | | |
|---|---|---|
| | C | H |
| Theoretical (%) | 70.43 | 8.69 |
| Experimental (%) | 69.98 | 8.72 |

Furthermore, a triene type component was identified and confirmed from the products showing signals in ¹H-NMR spectrum (in deuterated methanol at 25 degree C) at δ value of 1.25 ppm (the methylene group hydrogens in a long chain alkyl group), 1.58 ppm (the second methylene group hydrogens in a long chain alkyl group counting from the aromatic section), 2. 00 ppm (the methylene group hydrogen connected to the vinyl group), 2. 56 ppm (the methylene group hydrogens directly connected to the aromatic group) , 3.13-3.69 ppm (the hydrogens connected to the C2, C3, C4, C5 and C6 carbon atoms in the oligo chain), 4.82 ppm (the anomer hydrogen connected to the C1 carbon in the oligo chain), 4.95-5.85 ppm (the vinyl group hydrogen), 6. 79, 6. 80-6. 89 ppm and 7.19-7.20 ppm (the hydrogen connected to the aromatic ring). The ¹H-NMR spectrum of the triene type component obtained is shown in Figure 4.

### Example 1

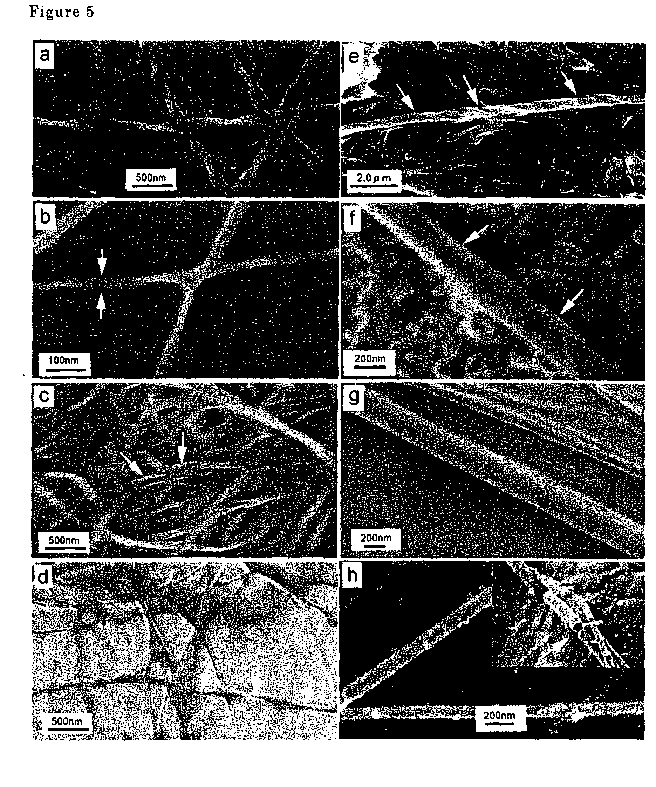

3 mg of the saturated component obtained in Production Example 2 were weighed into a flask, 50 ml of water was added to the contents and a mantle heater was used to heat the contents to boil the mixture and to dissolve. The mantle heater heating rate was adjusted slowly, and the mixture was allowed to cool to room temperature at a cooling rate of 0.2 degree C/minute. The mixture was allowed to stand for a day at room temperature. The aqueous solution containing the fibrous material obtained was collected and was evaluated using a transmission type electron microscope. The presence of helical ribbon self-aggregates several tens of nm thick, several hundreds of nm in pitch and several hundreds of microns long was confirmed. A transmission type electron microscope photograph is shown in Figure 5a.

### Example 2

A total of 3 mg of the saturated type component and the monoene type component obtained in Production Example 2 in a weight ratio of 9:1 was weighed and dissolved in methanol. The methanol was allowed to evaporate and dry, and 50 ml of water was added to the solid residue. A mantle heater was used to heat, boil and dissolve the solids. The mantle heater heating rate was adjusted slowly, and the mixture was allowed to cool to room temperature at a cooling rate of 0.2 degree C/minutes. The mixture was allowed to stand for a day at room temperature. The aqueous solution containing the fibrous material obtained was collected and was evaluated using a transmission type electron microscope. The presence of nanoscale self-aggregated structures similar to the one obtained in Example 1 several tens of nm thick, several hundreds of nm in pitch and several hundreds of microns in length was confirmed. A transmission type electron microscope photograph is shown in Figure 5b.

### Example 3

A nanoscale self-aggregated structure was obtained using the self aggregation process completely identical to the one described in Example 2 with the exception of using the saturated type component and the monoene type component in a weight ratio of 8:2 in place of 9:1. The presence of helical ribbon self-aggregates, the same as the nanoscale self-aggregated structure obtained in Example 1 having, that were several tens of nm thick, several hundreds of nm in pitch and several hundreds of microns in length was confirmed when the aqueous solution containing the fibrous material obtained was collected and evaluated using a transmission type electron microscope. A transmission type electron microscope photograph is shown in Figure 5c.

### Example 4

A nanoscale self-aggregated structure was obtained using the self aggregation process completely identical to the one described in Example 2 with the exception of using the saturated type component and a monoene type component in a weight ratio of 5:5 in place of 9:1. The presence of loosely coiled ribbon self-aggregates several tens of nm thick, about 500 nm in pitch and several hundreds of microns in length was confirmed when the aqueous solution containing the fibrous material obtained was collected and evaluated using a transmission type electron microscope. A transmission type electron microscope photograph is shown in Figure 5d.

### Example 5

A nanoscale self-aggregated structure was obtained using the self aggregation process completely identical to the one described in Example 2 with the exception of using the saturated type component and a monoene type component in a weight ratio of 2:8 in place of 9:1. The presence of tightly coiled ribbon self-aggregates several tens of nm thick, 500 of nm in pitch and several hundreds of microns in length was confirmed when the aqueous solution containing the fibrous material obtained was collected and evaluated using a transmission type electron microscope. A transmission type electron microscope photograph is shown in Figure 5e.

### Example 6

A nanoscale self-aggregated structure was obtained using the self aggregation process completely identical to the one described in Example 2 with the exception of using the saturated type component and monoene type component in a weight ratio of 5:5 in place of 9:1. The existence of a tubular morphology having slight helical traces several tens of nm thick, about 500 nm in pitch and several hundreds of microns in length was confirmed when the aqueous solution containing the fibrous material obtained was collected and evaluated using a transmission type electron microscope. A transmission type electron microscope photograph is shown in Figure 5f.

### Example 7

Three milligrams of the monoene type component obtained in Production Example 2 was weighed into a flask, and 50 ml of water was added. A mantle heater was used to heat, boil and dissolve the solids. The mantle heater heating rate was adjusted slowly, and the mixture was allowed to cool to room temperature at a cooling rate of 0.2 degree C/minutes. The mixture was allowed to stand for a day at room temperature. The aqueous solution containing the fibrous material obtained was collected and was evaluated using a transmission type electron microscope. The presence of perfectly tubular self-aggregates several tens of nm thick and several hundreds of microns in length was confirmed. A transmission type electron microscope photograph is shown in Figure 5g.

### Reference Example 1

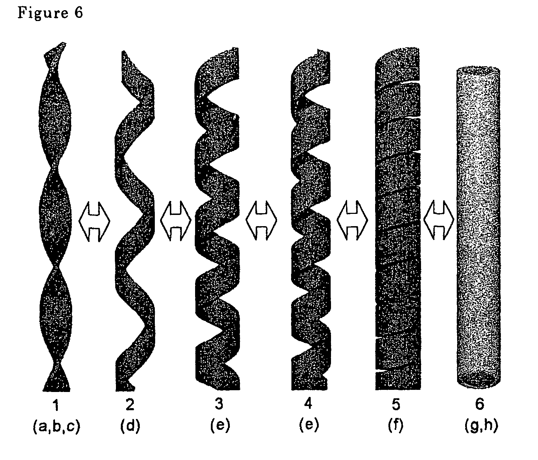

A nanoscale self-aggregated structure was obtained using the same self aggregation method described in Example 2 with the exception of using the O-glycoside type oligolipid (the hydrocarbon group segment in natural cardanol) obtained in Production Example 1 in place of the O-glycoside type oligolipid used in Example 2. The aqueous solution obtained containing a fibrous material was collected and evaluated using a transmission type electron microscope. The presence of a perfectly tubular self-aggregate several tens of nm thick and several hundreds of microns long was confirmed. A transmission type electron microscope photograph is shown in Figure 5h.
A synthetic lipid prepared using naturally produced cardanol (a mixture of four types) undergoes self aggregation to yield nanotubes. The results of a self aggregation morphology study of individual components, the saturated type component and the monoene component, indicated that the former yielded a twisted ribbon (Example 1) and the latter yielded tightly coiled ribbon or nanotube morphology (Example 7). When these two types are mixed, the fact that tubular shapes ranging from twisting to coiling shapes can be formed was discovered (Examples 2-6). This type of tubular morphology change is shown in Figure 6. (In the figures, the symbols inside parentheses correspond to the Figure 5 symbols.)

Based on this study, the diene type component and triene type component do not appear to contribute toward nanotube formation from a mixture. An experimental result based on differential scanning calorimetric analysis (not reported here) indicates that diene type and triene type components exist in water at room temperature in the form of liquid crystals and are not associated with nanotubes existing as a solid phase. However, a potential for nanotubes containing traces of the components exists but is difficult to prove experimentally. Therefore, a mixture of two species, the saturated and monoene type components, can be said to control the tubular morphology.

## Claims

1. A fibrous nanoscale self-aggregate comprising O-glycoside type oligolipid represented by the general formula below (Chemical Formula 1) 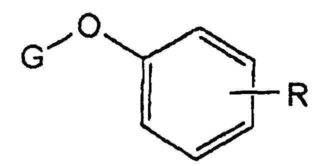 (wherein G represents a glycosyl group and R represents a hydrocarbon group containing 12 to 18 carbon atoms), wherein said O-glycoside type oligolipid comprises at least two kinds of O-glycoside type oligolipids having different said structure, wherein the proportion of the two major kinds of said O-glycoside type oligolipid being at least 80% by weight of said O-glycoside type oligolipid, or comprises an O-glycoside type oligolipid having one type of said structure.

2. The fibrous nanoscale self-aggregate as in Claim 1, wherein R in Chemical Formula 1 is in meta position to the -O-G group.

3. The fibrous nanoscale self-aggregate as in Claim 1 or 2, wherein said O-glycoside type glycolipid is a mixture of two kinds of 0-glycoside type glycolipids.

4. The fibrous nanoscale self-aggregate as in any one of Claims 1 to 3, wherein said at least two kinds of O-glycoside type oligolipid have different aforementioned hydrocarbon groups.

5. The fibrous nanoscale self-aggregate as in Claim 4, wherein said hydrocarbon groups have different degrees of saturation.

6. The fibrous nanoscale self-aggregate as in Claim 5, wherein said hydrocarbon group is saturated or monoene.

7. A process for producing a fibrous nanoscale self-aggregate comprising an O-glycoside type oligolipid having a structure represented by the general formula below (Chemical Formula 1) 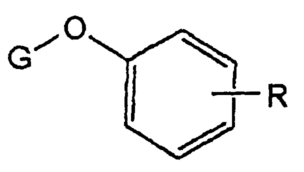 (wherein G represents a glycosyl group and R represents a hydrocarbon group containing 12 to 18 carbon atoms) which comprises a step of dispersing O-glycoside type oligolipid in an aqueous medium, wherein said O-glycoside type oligolipid comprises at least two kinds of O-glycoside type oligolipids having different said structure, wherein the proportion of the two major kinds of said O-glycoside type oligolipid being at least 80% by weight of said O-glycoside type oligolipid, or comprises an O-glycoside type oligolipid having one type of said structure.

8. The process as in Claim 7, wherein R in Chemical Formula 1 is in meta position to the -O-G group.

9. The process as in Claim 7 or 8, wherein said O-glycoside type glycolipid is a mixture of two kinds of O-glycoside type glycolipids.

10. The process described in any one of Claims 7 to 9, wherein said at least two kinds of O-glycoside type oligolipid have different aforementioned hydrocarbon groups.

11. The process as in Claim 10, wherein said hydrocarbon groups have different degrees of saturation.

12. The process as in Claim 11, wherein said hydrocarbon group is saturated or monoene.
